# EUROPÄISCHE PATENTSCHRIFT

(11) **EP 3 073 264 B1**
(45) Veröffentlichungstag und Bekanntmachung des Hinweises auf die Patenterteilung: **13.09.2017**
(21) Anmeldenummer: 16159932.9
(22) Anmeldetag: 11.03.2016
(51) Int. Cl.: G01N 33/487, C12N 1/06, C12Q 1/68

(54) **VERFAHREN ZUM BETREIBEN EINER SEQUENZIERVORRICHTUNG**
METHOD FOR OPERATING A SEQUENCING DEVICE
PROCEDE POUR FAIRE FONCTIONNER UN SEQUENCEUR

(30) Priorität: 25.03.2015 DE 102015205435
(43) Veröffentlichungstag der Anmeldung: 28.09.2016
(73) Patentinhaber: ROBERT BOSCH GMBH, 70442 Stuttgart (DE)
(72) Erfinder: Hoffmann, Jochen, 71229 Leonberg (DE); Lemuth, Karin, 70195 Stuttgart (DE)

(56) Entgegenhaltungen:
- DE-A1-102013 217 694
- US-A1- 2006 240 543
- US-A1- 2010 292 101

## Beschreibung

### Stand oder technik

Die Erfindung geht aus von einem Verfahren nach Gattung des unabhängigen Anspruchs. Die Entschlüsselung des genetischen Codes wird auch als DNA-Sequenzierung bezeichnet und wird in der Wissenschaft, der Medizin und der Forensik häufig angewendet. Für die Sequenzierung ist bisher eine umfangreiche Probenvorbereitung, wie Lyse und Aufreinigung unerlässlich, um ausreichend reine Nukleinsäuren für die Sequenzierung zu erhalten.

Aus US 2010/0292101, US 2006/0240543 A1 und DE 10 2013 217 694 A1 sind mikrofluidische Vorrichtungen und Verfahren zur Analyse von Molekülen beziehungsweise Zellen bekannt.

### Offenbarung der Erfindung

Vor diesem Hintergrund wird mit dem hier vorgestellten Ansatz ein Verfahren zum Betreiben einer Sequenziervorrichtung gemäß dem Hauptanspruch vorgestellt. Durch die in den abhängigen Ansprüchen aufgeführten Maßnahmen sind vorteilhafte Weiterbildungen und Verbesserungen des im unabhängigen Anspruch angegebenen Verfahrens möglich.

Es wird eine Sequenziervorrichtung verwendet, die zumindest einen Sequenzierkanal aufweist, der einen ersten Spalt mit einem zweiten Spalt fluidisch verbindet, wobei der Sequenzierkanal im Bereich des ersten Spalts als eine Kavität ausgebildet ist und im Bereich des zweiten Spalts als eine Pore ausgebildet ist, wobei die Pore einen geringeren Querschnitt aufweist, als die Kavität.
Unter einer Sequenziervorrichtung kann eine Vorrichtung zum Bestimmen einer DNA-Sequenz verstanden werden. Die DNA-Sequenz kann durch eine herkömmliche Auswerteeinrichtung ausgewertet werden. Ein Spalt kann ein Kanal zum Führen eines Mediums beziehungsweise zum Führen von mehreren Medien sein. Insbesondere kann der erste Spalt durch einen Abstand zwischen einem Grundkörper der Vorrichtung und einem ersten flächigen Substrat ausgebildet sein. Der zweite Spalt kann durch einen Abstand zwischen dem Grundkörper und einem zweiten flächigen Substrat ausgebildet sein. Dabei können die Substrate auf gegenüberliegenden Seiten des Grundkörpers angeordnet sein. Die Substrate können im Wesentlichen parallel zueinander angeordnet sein. Ein Sequenzierkanal kann den Grundkörper durchbrechen oder öffnen. Eine Kavität kann als (breite) Vertiefung in einem Grundkörper mit einem fluidischen Durchgang in die (schmale) Pore bezeichnet werden, wobei die Pore ebenfalls eine Öffnung des Grundkörpers bildet. Die Kavität kann auch als Well bezeichnet werden. Der Querschnitt der Pore ist (wesentlich) geringer als der Querschnitt der Kavität, beispielsweise um eine Größenordnung kleiner als der Querschnitt der Kavität. Unter einem Querschnitt kann vorliegend eine Querschnittsfläche oder ein Abstand zwischen zwei gegenüberliegenden Rändern der Kavität und/oder der Pore im Bereich der Bildung des Querschnitts verstanden werden. Der Querschnitt kann auch einen Durchmesser des Sequenzierkanals bilden, wenn der Sequenzierkanal eine kreisförmige Grundfläche aufweist. Die Pore kann dabei eine Länge aufweisen, die im Wesentlichen einer Tiefe der Kavität entspricht.

Bei dem hier verwendeten beispielhaften Einzelmolekülsequenzierer entfällt eine klonale Amplifikation der zu sequenzierenden DNA-Moleküle beziehungsweise Nukleinsäuren, wodurch kein Sequencing-Bias auftritt. Ebenso wird keine Bridge-Amplification benötigt. Zusätzlich kann auf eine aufwendige Probenvorbereitung verzichtet werden.

Es wird eine mikrofluidische Umgebung verwendet, welche es erlaubt, Zellen einer Probenlösung individuell aufzuschließen, die DNA aufzureinigen und diese direkt im Anschluss in nachgeschalteten Poren individuell zu sequenzieren.

Das hier verwendete System ermöglicht eine echte Einzelzellsequenzierung. Dadurch wird es möglich, unter Verwendung mehrerer Sequenzierkanäle die genetische Heterogenität einer Zellen enthaltenden Probenlösung zu bestimmen. Es ergibt sich eine hohe Sensitivität des Gesamtsystems, da eine Aufreinigung beispielsweise über eine Säule, mit hohen Aufreinigungsverlusten bei kleinen DNA-Konzentrationen, entfällt.

Das hier verwendete System erlaubt ferner die Analyse kleinster Zellmengen, da jede Zelle für sich in einem Well des Microwell-Arrays gesondert aufgeschlossen und der Nanoporensequenzierung zugeführt werden kann.
Es ist keine gesonderte Probenaufreinigung für eine Sequenzierung mehr notwendig, alle einzelnen Schritte beziehungsweise Einzelverfahren können in einer einzigen mikrofluidischen Umgebung abgebildet und durchgeführt werden. Dadurch sinken die Fehleranfälligkeit des Gesamtsystems und der zeitliche Aufwand.

Bei dem hier verwendeten System ist eine DNA-Kontamination der zu sequenzierenden DNA bzw. Zellen ausgeschlossen, da die Zelllysate automatisiert und integriert in den eigentlichen Sequenzierprozess eingespeist werden, wodurch DNA-Verschleppungen und Kontaminationen vermieden werden, die das Sequenzierergebnis negativ beeinflussen könnten.
Die Kavität kann dazu ausgebildet sein, eine einzelne Zelle aufzunehmen. Dazu kann die Kavität so klein sein, dass gerade eine einzige Zelle hineinpasst. Insbesondere kann die Kavität einen Querschnitt zwischen einem Mikrometer und dreihundert Mikrometern aufweisen. Insbesondere kann die Kavität einen Querschnitt zwischen drei Mikrometern und 30 Mikrometern aufweisen.

Der erste Spalt und alternativ oder ergänzend der zweite Spalt können eine Spaltbreite zwischen zwei Mikrometern und einem Millimeter aufweisen. Insbesondere kann der erste Spalt und alternativ oder ergänzend der zweite Spalt eine Spaltbreite zwischen fünf Mikrometern und 500 Mikrometern aufweisen. Abhängig von den zum Vorbereiten des Sequenzierens und zum Sequenzieren selber verwendeten Medien kann die Spaltbreite angepasst werden. Insbesondere kann die Spaltbreite für eine laminare Strömung in den Spalten optimiert sein.
In der Pore kann eine Elektrophoresestrecke angeordnet sein. Dadurch kann die hier vorgestellte Sequenziervorrichtung direkt für eine Elektrophorese verwendet werden.
Die Sequenziervorrichtung kann zumindest einen weiteren Sequenzierkanal aufweisen. Insbesondere kann eine Vielzahl von Sequenzierkanälen zu einer Matrix angeordnet sein. Insbesondere kann die Matrix eine Dichte zwischen 1x10³ und 25x10⁶ Sequenzierkanälen pro Quadratzentimeter aufweisen. Durch viele Sequenzierkanäle können viele Sequenzierungen gleichzeitig durchgeführt werden. Erfindungsgemäß wird ein Verfahren zum Betreiben einer mikrofluidischen Sequenziervorrichtung vorgestellt, die zumindest einen mikrofluidischen Sequenzierkanal aufweist, der einen ersten mikrofluidischen Spalt mit einem zweiten mikrofluidischen Spalt fluidisch verbindet, wobei der Sequenzierkanal im Bereich des ersten Spalts als eine Kavität ausgebildet ist und im Bereich des zweiten Spalts als eine Pore ausgebildet ist, wobei die Pore einen geringeren Querschnitt aufweist, als die Kavität, wobei das Verfahren die folgenden Schritte aufweist:
Einleiten einer Probenlösung in den ersten Spalt, um eine Zelle in die Kavität einzubringen;
Lysieren der Zelle in ihre Zellbestandteile, um zelluläre DNA der Zelle freizusetzen;
Spülen des ersten Spalts, um unerwünschte Zellbestandteile der Zelle aus der Kavität und/oder dem ersten Spalt zu entfernen und die DNA in der Kavität zu isolieren;
Füllen des ersten Spalts und des zweiten Spalts mit einem Sequenzierpuffer; und
Sequenzieren der DNA in der Pore.

Als Probenlösung kann eine wässrige Lösung, die Zellen enthält, eingeleitet werden.

Im Schritt des Einleitens kann ferner eine Zentrifugation, eine Sedimentation und alternativ oder ergänzend eine Vakuumbehandlung durchgeführt werden, um die Zelle in die Kavität einzubringen. Dadurch können die Einzelzellen ortsaufgelöst sequenziert und die Sequenziervorgang beschleunigt werden.

Im Schritt des Lysierens kann z. B. eine chemische Lyse, eine enzymatische Lyse, eine elektrische Lyse, eine Ultraschalllyse und alternativ oder ergänzend eine thermische Lyse durchgeführt werden, um die zelluläre DNA freizusetzen. In der hier vorgestellten Sequenziervorrichtung können verschiedene Lyseverfahren durchgeführt und/oder kombiniert werden. Damit können verschiedenen Zellen lysiert werden.

Im Schritt des Lysierens kann eine organische Phase (d. h. Flüssigkeit) in den ersten Spalt eingebracht werden. Die organische Phase kann insbesondere mit einer laminaren Strömung in den ersten Spalt eingebracht werden. Die organische Phase kann eine geringe Löslichkeit in der wässrigen Lösung aufweisen. Durch die laminare Strömung können Verwirbelungen vermieden werden.

Im Schritt des Spülens kann eine Spülflüssigkeit in den ersten Spalt eingebracht werden. Die Spülflüssigkeit kann insbesondere mit einer laminaren Strömung in den ersten Spalt eingebracht werden. Die Spülflüssigkeit kann eine Lysierflüssigkeit gut entfernen beziehungsweise verdrängen. Die Spülflüssigkeit kann selbst gut aus dem Spalt entfernt werden. Durch die laminare Strömung können Verwirbelungen vermieden werden.
Im Schritt des Sequenzierens kann eine elektrische Spannung zwischen einem den ersten Spalt begrenzenden ersten Substrat und einem den zweiten Spalt begrenzenden zweiten Substrat angelegt werden, um die DNA in der Pore zu sequenzieren. Durch ein resultierendes elektrisches Feld kann die DNA unter Verwendung der Elektrophorese sequenziert werden.
Dieses Verfahren kann beispielsweise in Software oder Hardware oder in einer Mischform aus Software und Hardware beispielsweise in einem Steuergerät implementiert sein.

Ausführungsbeispiele der Erfindung sind in den Zeichnungen dargestellt und in der nachfolgenden Beschreibung näher erläutert. Es zeigt:
Fig. 1 eine Darstellung einer Sequenziervorrichtung;
Fig. 2 ein Ablaufdiagramm eines Verfahrens zum Betreiben einer Sequenziervorrichtung gemäß einem Ausführungsbeispiel;
Fig. 3 eine Darstellung eines Einleitens einer Probenlösung in eine Sequenziervorrichtung gemäß einem Ausführungsbeispiel;
Fig. 4 eine Darstellung eines Lysierens von Zellen in einer Sequenziervorrichtung gemäß einem Ausführungsbeispiel;
Fig. 5 eine Darstellung von isolierter DNA in einer Sequenziervorrichtung gemäß einem Ausführungsbeispiel; und
Fig. 6 eine Darstellung eines Sequenzierens von DNA in einer Sequenziervorrichtung gemäß einem Ausführungsbeispiel.
In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

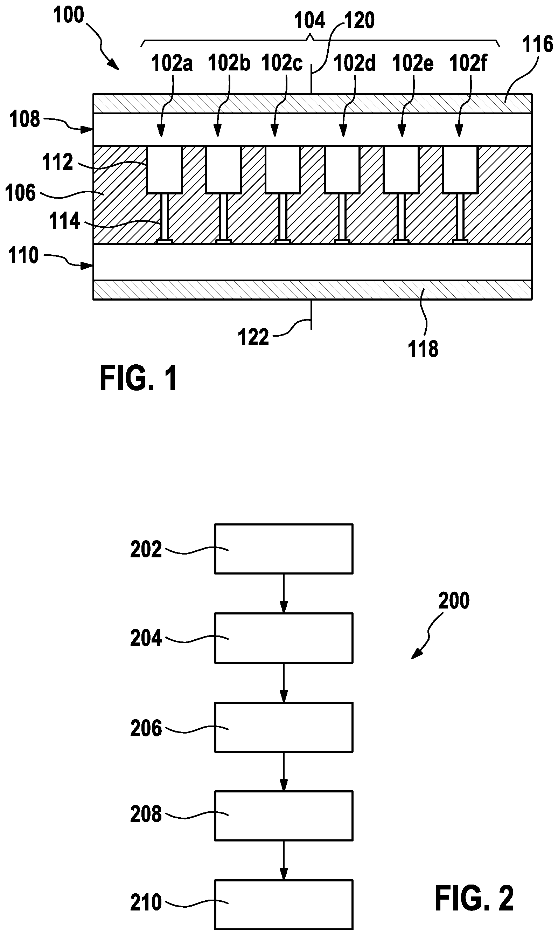

Fig. 1 zeigt eine Darstellung einer Sequenziervorrichtung 100. Die Sequenziervorrichtung 100 weist eine Mehrzahl von Sequenzierkanälen 102 auf. In der hier gewählten Darstellungsebene sind ein erster Sequenzierkanal 102a, ein zweiter Sequenzierkanal 102b, ein dritter Sequenzierkanal 102c, ein vierter Sequenzierkanal 102d, ein fünfter Sequenzierkanal 102e und ein sechster Sequenzierkanal 102f nebeneinander dargestellt. Die Sequenzierkanäle 102 sind gleichartig ausgeführt. Die Sequenzierkanäle 102 sind zu einer Matrix 104 angeordnet. Die Sequenzierkanäle 102 sind in einem plattenförmigen Grundkörper 106 ausgebildet. Jeder der Sequenzierkanäle 102 stellt eine fluidische Verbindung zwischen einem ersten Spalt 108 auf einer ersten Seite des Grundkörpers 106 und einem zweiten Spalt 110 auf einer gegenüberliegenden zweiten Seite des Grundkörpers 106 dar.
Der Sequenzierkanal 102 ist im Bereich des ersten Spalts 108 als eine Kavität 112 ausgebildet. Im Bereich des zweiten Spalts 110 ist der Sequenzierkanal 102 als eine Pore 114 ausgebildet. Dabei weist die Pore 114 einen (wesentlich) geringeren Querschnitt auf, als die Kavität 112. Insbesondere ist die Kavität 112 dazu ausgebildet, eine einzige zu sequenzierende Zelle aufzunehmen. Beispielsweise ist der Querschnitt oder Durchmesser der Kavität um das 100-Fache bis 100.000-Fache größer als der Querschnitt oder Durchmesser der Pore.

Der erste Spalt 108 ist zwischen dem Grundkörper 106 und einem Deckelsubstrat 116 ausgebildet und weist eine Spaltbreite auf, die dazu ausgebildet ist, eine laminare Strömung im ersten Spalt 108 zu ermöglichen.
Der zweite Spalt 110 ist hier zwischen dem Grundkörper 106 und einem Bodensubstrat 118 ausgebildet. Der zweite Spalt 110 weist ebenfalls eine Spaltbreite auf, die dazu ausgebildet ist, eine laminare oder nicht-laminare Strömung im zweiten Spalt 108 zu ermöglichen.
Das Deckelsubstrat 116 weist einen ersten elektrischen Anschluss 120 zum elektrischen Kontaktieren des Deckelsubstrats 116 auf.

Das Bodensubstrat 118 weist einen zweiten elektrischen Anschluss 122 zum elektrischen Kontaktieren des Bodensubstrats 118 auf.

Zwischen den beiden elektrischen Kontakten 120, 122 kann eine elektrische Spannung zum Erzeugen eines elektrischen Felds zwischen dem Deckelsubstrat 116 und dem Bodensubstrat 118 angelegt werden.

Es ist ein Querschnitt durch einen Mikrowell-Nanoporen Hybrid 100 dargestellt. Das Mikrowell-Array 104 weist zur Oberfläche des Mikrowell-Arrays 104 hin geöffnete Kavitäten 112 auf. Die Kavitäten 112 können als Wells 112 bezeichnet werden. An die Unterseite der Wells 112 ist jeweils eine Pore 114 beziehungsweise Nanopore 114 angeschlossen.

Zwischen der Mikrowell-Oberfläche und einem Deckelsubstrat 116 besteht ein mikrofluidischer Spalt 108 mit Abmessungen von zwei bis 1000 µm, bevorzugt fünf bis 500 µm. Zwischen der Unterseite des Mikrowell-Arrays 104 und dem Bodensubstrat 118 besteht ebenfalls ein mikrofluidischer Spalt 110 mit Abmessungen von zwei bis 1000 µm, bevorzugt fünf bis 500 µm. Der Abstand ist so gewählt, dass eine laminare Durchströmung begünstigt ist. Zwischen Deckelsubstrat 116 und Bodensubstrat 118 kann ein elektrisches Feld appliziert werden.

Bei dem hier vorgestellten Mikrowell-Array 104 können die Abmessungen eines Wells 112 zwischen 500 nm und 300 µm, bevorzugterweise zwischen einem µm und 30 µm liegen. Die Dichte der Wells 112 auf dem Mikrowell-Array 104 kann zwischen 1x10³ und 25x10⁶ Wells 112 pro cm² liegen.

Der hier vorgestellte Ansatz kann für analytische Systeme insbesondere für mikrofluidische Lab-on-Chip-Systeme zur Umweltanalytik oder medizinischen Diagnostik verwendet werden.

Fig. 2 zeigt ein Ablaufdiagramm eines Verfahrens 200 zum Betreiben einer Sequenziervorrichtung gemäß einem Ausführungsbeispiel. Das Verfahren 200 kann auf einer Sequenziervorrichtung, wie sie in Fig. 1 dargestellt ist, ausgeführt werden. Dazu ist die Sequenziervorrichtung mit einer Vorrichtung zum Betreiben der Sequenziervorrichtung verbunden. Insbesondere ist die Sequenziervorrichtung als einmal verwendbare Kartusche ausgeführt, die über Schnittstellen mit der Vorrichtung zum Betreiben verbunden ist.

Das Verfahren 200 weist einen Schritt 202 des Einleitens, einen Schritt 204 des Lysierens, einen Schritt 206 des Spülens, einen Schritt 208 des Füllens und einen Schritt 210 des Sequenzierens auf. Im Schritt 202 des Einleitens wird eine Probenlösung in den ersten Spalt eingeleitet, um zumindest in eine der Kavitäten eine einzelne Zelle einzubringen. Beispielsweise wird zum Einleiten ein Unterdruck in dem ersten Spalt aufgebaut, um die Probenlösung in den ersten Spalt und damit in die Kavitäten zu saugen. Ebenso kann die Probenlösung durch einen Überdruck in den ersten Spalt eingepresst werden. Alternativ kann die Probenlösung durch den Kapillareffekt in den Spalt gezogen werden. Im Schritt 204 des Lysierens wird die zumindest eine Zelle innerhalb ihrer Kavität in ihre Zellbestandteile zerlegt, um zelluläre DNA der Zelle freizusetzen. Das Lysieren kann auf verschiedene Weisen erfolgen. Beispielsweise kann im Schritt 204 des Lysierens eine chemische Lyse, eine enzymatische Lyse, eine elektrische Lyse, eine Ultraschalllyse und alternativ oder ergänzend eine thermische Lyse durchgeführt werden, um die zelluläre DNA freizusetzen. Im Schritt 206 des Spülens wird der erste Spalt gespült, um unerwünschte Zellbestandteile der Zelle aus der Kavität und alternativ oder ergänzend aus dem ersten Spalt zu entfernen und die DNA in der Kavität zu isolieren. Insbesondere kann ein Spülfluid durch den ersten Spalt geleitet werden, das selektiv alle Zellbestandteile außer der DNA aus der Kavität entfernt. Im Schritt 208 des Füllens werden der erste Spalt und der zweite Spalt mit einem Sequenzierpuffer gefüllt. Wie im Schritt 202 des Einleitens kann der Sequenzierpuffer in die Spalte gesaugt beziehungsweise gezogen und/oder gedrückt werden. Im Schritt 210 des Sequenzierens wird die DNA in der Pore sequenziert.

Im Schritt 202 kann ferner eine Zentrifugation, eine Sedimentation und alternativ oder ergänzend eine Vakuumbehandlung durchgeführt werden, um die Zelle in die Kavität einzubringen.

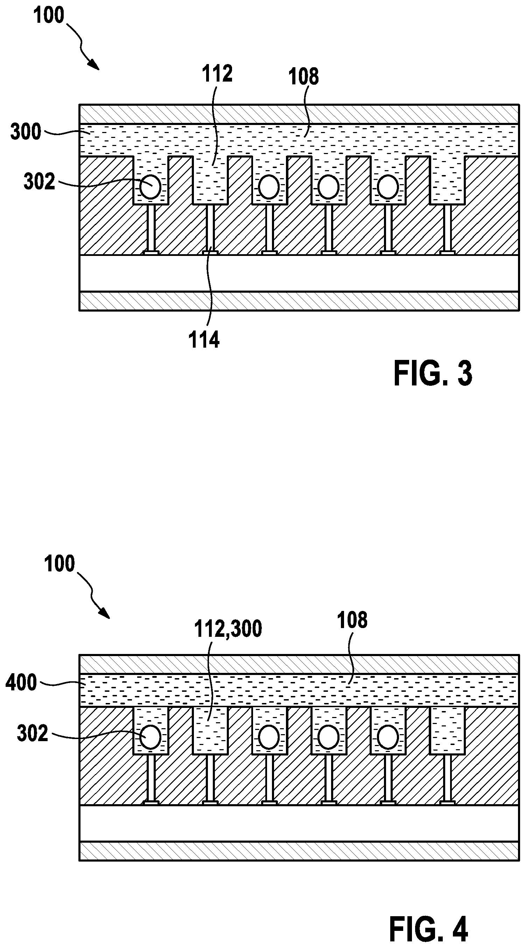

Fig. 3 zeigt eine Darstellung eines Einleitens einer Probenlösung 300 in eine Sequenziervorrichtung 100 gemäß einem Ausführungsbeispiel. Die Sequenziervorrichtung 100 entspricht dabei im Wesentlichen der Sequenziervorrichtung in Fig. 1. Das Einleiten repräsentiert einen Schritt eines Verfahrens zum Betreiben der Sequenziervorrichtung, wie es in Fig. 2 beschrieben ist.

Hier wird eine wässrige Lösung 300, die Zellen 302 enthält, in den ersten Spalt 108 eingeleitet. Die Kavitäten 112 werden dabei ebenfalls mit der wässrigen Lösung 300 benetzt. Durch eine Zentrifugation, eine Sedimentation und alternativ oder ergänzend eine Vakuumbehandlung werden die Zellen 302 in die Kavitäten 112 eingebracht. In eine Kavität 112 passt dabei jeweils nur eine Zelle 302. Dadurch sind die einzelnen Zellen 302 voneinander isoliert.

Es ist ein Ausführungsbeispiel für eine Aufreinigung beziehungsweise Purifikation der zellulären DNA dargestellt. Hier kommt eine miniaturisierte Phenol-Chloroform-Extraktion zum Einsatz. Hierfür werden die zu analysierenden Zellen 302 in Wasser beziehungsweise eine Zellen 302 enthaltende Probenlösung 300 in den Chip 100 eingebracht.

Fig. 4 zeigt eine Darstellung eines Lysierens von Zellen 302 in einer Sequenziervorrichtung 100 gemäß einem Ausführungsbeispiel. Die Sequenziervorrichtung 100 entspricht dabei im Wesentlichen der Sequenziervorrichtung in Fig. 1. Das Lysieren repräsentiert einen Schritt eines Verfahrens zum Betreiben der Sequenziervorrichtung, wie es in Fig. 2 beschrieben ist.

Dabei wird der erste Spalt 108 mit einer Lysierflüssigkeit 400 gefüllt. Die Lysierflüssigkeit 400 bewirkt eine Lyse der Zellen 302, dabei werden die Zellen in ihre Zellbestandteile zerlegt. Alternativ können die Zellen durch Anlegen eines elektrischen Feldes zwischen 120 und 122 elektrisch lysiert werden. Die Lysierflüssigkeit 400 beispielsweise dringt aufgrund ihrer Oberflächenspannung nur unwesentlich in die Kavitäten 112 ein. In jeder der Kavitäten 112 bleibt ein Rest der wässrigen Lösung 300 zurück.

Anschließend an das Einbringen wird der mikrofluidische Spalt 108 mit einer organischen Phase 400, hier Phenol im Verhältnis 1:1 gefüllt. Die in der wässrigen Lösung 300 enthaltene DNA, Zellfragmente und Zellinnereien werden aufgrund ihrer unterschiedlichen Löslichkeiten aufgetrennt. Die DNA verbleibt in der wässrigen Phase 300, alle anderen Bestandteile gehen durch Diffusion in die organische Phase 400 über. Durch anschließendes Ausspülen der organischen Phase 400 beispielsweise mit Chloroform und Isoamylalkohol beispielsweise im Verhältnis 24:1 werden diese Bestandteile aus dem Chip 100 entfernt.

Mit anderen Worten zeigen die Figuren 3 und 4 Abbildungen als Zwischenstadien in einem Ablauf einer Probenvorbereitung für eine Nanoporen-Sequenzierung gemäß dem hier vorgestellten Ansatz.

Für die Probenvorbereitung wird in Fig. 3 eine Zellen enthaltende Probenlösung in den mikrofluidischen Spalt eingeleitet. Die Zellen werden mittels Zentrifugation, Sedimentation oder vakuumunterstützt in die Wells eingebracht. Anschließend werden in Fig. 4 die Zellen lysiert. Hierfür können bekannte Methoden, wie eine chemische Lyse, eine enzymatische Lyse, eine elektrische Lyse, eine Ultraschall Lyse und/oder eine thermische Lyse eingesetzt werden.

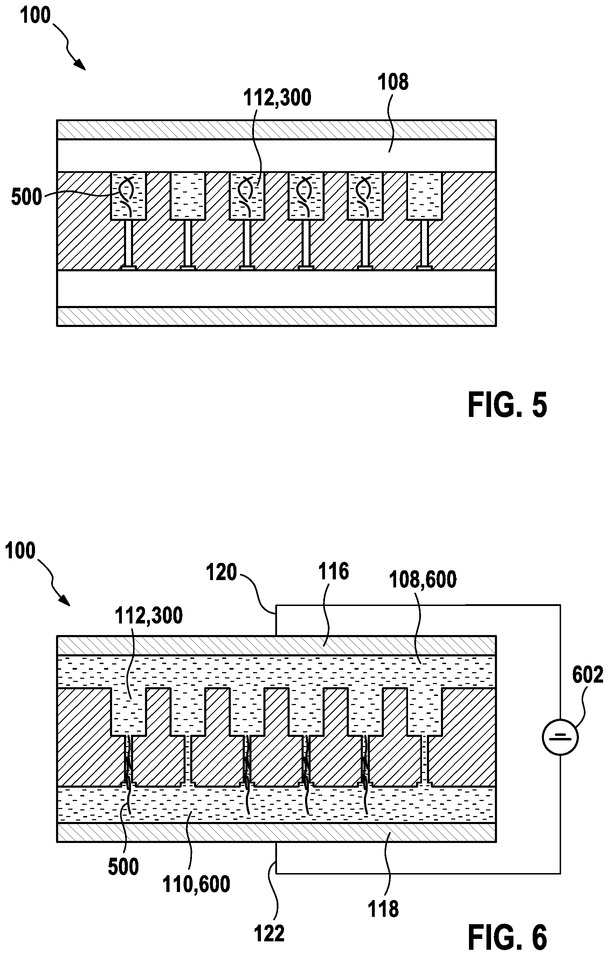

Fig. 5 zeigt eine Darstellung von isolierter DNA 500 in einer Sequenziervorrichtung 100 gemäß einem Ausführungsbeispiel. Die Sequenziervorrichtung 100 entspricht dabei im Wesentlichen der Sequenziervorrichtung in Fig. 1. Hier ist die Lysierflüssigkeit beziehungsweise eine nachfolgend in den ersten Spalt 108 eingebrachte Spülflüssigkeit aus Fig. 4 aus dem ersten Spalt 108 entfernt. In jeder Kavität 112 ist der Rest der wässrigen Lösung 300 mit der DNA 500 angeordnet.

Es ist der Chip 100 nach der Purifikation dargestellt. Hierbei ist die zelluläre DNA 500 in den jeweiligen Wells 112 isoliert in einer wässrigen Phase 300 vorhanden.

Fig. 6 zeigt eine Darstellung eines Sequenzierens von DNA 500 in einer Sequenziervorrichtung 100 gemäß einem Ausführungsbeispiel. Die Sequenziervorrichtung 100 entspricht dabei im Wesentlichen der Sequenziervorrichtung in Fig. 1. Das Sequenzieren repräsentiert einen Schritt eines Verfahrens zum Betreiben der Sequenziervorrichtung, wie es in Fig. 2 beschrieben ist.

Hier ist ein Sequenziermedium 600 in den ersten Spalt 108 und den zweiten Spalt 110 eingebracht. Die Poren 114 sind ebenfalls mit dem Sequenziermedium 600 gefüllt. In den Kavitäten 112 ist dabei zunächst die wässrige Lösung 300 angeordnet. Die wässrige Lösung vermischt sich mittels Diffusion mit dem Sequenziermedium. Als Resultat sind die Kavitäten mit dem Sequenziermedium gefüllt (die wässrige Lösung ist rausverdünnt). Eine Gleichspannungsquelle 602 ist zwischen die elektrischen Anschlüsse 120, 122 geschaltet. Dadurch wird zwischen dem Deckelsubstrat 116 und dem Bodensubstrat 118 ein elektrisches Feld ausgebildet. Das elektrische Feld zieht die DNA durch die Poren 114, wobei eine Wanderungsgeschwindigkeit der DNA 500 beziehungsweise Teilstücke der DNA 500 in dem Sequenziermedium 600 ausgewertet werden können.

Es ist ein Ausführungsbeispiel einer Sequenzierung dargestellt. Hierfür werden die mikrofluidischen Spalte 108, 110 mit einem Sequenzierpuffer 600 gefüllt. Die Poren 114 weisen eine Elektrophorese-Strecke auf, beispielsweise den gleichen Puffer 600mit dem auch die Spalte 108, 110 befüllt sind oder ein Agarosegel.

Wenn an das Deckelsubstrat 116 und das Bodensubstrat 118 eine Spannung angelegt wird, wird die zelluläre DNA 500 eines jeden Wells 112 durch die an die Wells 112 angeschlossenen Poren 114 geleitet.

Die Fragmentlänge der DNA 500 und/oder die Basen-genaue Detektion kann mit bekannten Methoden erfolgen. Beispielsweise kann die Detektion elektrisch, elektrochemisch oder optisch erfolgen. Abhängig von der Detektionsmethode können verschiedene Puffer 600 eingesetzt werden. beispielsweise kann 1 M KCl Salzlösung mit 10mMTris-HCl und 1mM EDTA (Ethylendiamintetraessigsäure), pH
8.0 bei Raumtemperatur oder 3M KCl Lösung pH 10.4, mit 1mM EDTA für die elektrische Detektion mittels einer Solid State Nanopore eingesetzt werden.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

## Patentansprüche

1. Verfahren (200) zum Betreiben einer mikrofluidischen Sequenziervorrichtung (100), die zumindest einen mikrofluidischen Sequenzierkanal (102) aufweist, der einen ersten mikrofluidischen Spalt (108) mit einem zweiten mikrofluidischen Spalt (110) fluidisch verbindet, wobei der Sequenzierkanal (102) im Bereich des ersten Spalts (108) als eine Kavität (112) ausgebildet ist und im Bereich des zweiten Spalts (110) als eine Pore (114) ausgebildet ist, wobei die Pore (114) einen geringeren Querschnitt aufweist, als die Kavität (112), wobei das Verfahren (200) die folgenden Schritte aufweist:
Einleiten (202) einer Probenlösung (300) in den ersten Spalt (108), um eine Zelle (302) in die Kavität (112) einzubringen;
Lysieren (204) der Zelle (302) in ihre Zellbestandteile, um zelluläre DNA (500) der Zelle (302) freizusetzen;
Spülen (206) des ersten Spalts (108), um unerwünschte Zellbestandteile der Zelle (302) aus der Kavität (112) und/oder dem ersten Spalt (108) zu entfernen und die DNA (500) in der Kavität (112) zu isolieren;
Füllen (208) des ersten Spalts (108) und des zweiten Spalts (110) mit einem Sequenzierpuffer (600); und
Sequenzieren (210) der DNA (500) in der Pore (114).

2. Verfahren (200) gemäß Anspruch 1, bei dem im Schritt (202) des Einleitens als Probenlösung (300) eine wässrige Lösung (300), die Zellen (302) enthält, eingeleitet wird.

3. Verfahren (200) gemäß einem der vorangegangenen Ansprüche, bei dem im Schritt des Einleitens (202) ferner eine Zentrifugation, eine Sedimentation und/oder eine Vakuumbehandlung durchgeführt wird, um die Zelle (302) in die Kavität (112) einzubringen.

4. Verfahren (200) gemäß einem der vorangegangenen Ansprüche, bei dem im Schritt (204) des Lysierens eine chemische Lyse, eine enzymatische Lyse, eine elektrische Lyse, eine Ultraschalllyse und/oder eine thermische Lyse durchgeführt wird, um die zelluläre DNA (500) freizusetzen.

5. Verfahren (200) gemäß einem der vorangegangenen Ansprüche, bei dem im Schritt (204) des Lysierens eine organische Phase (300) in den ersten Spalt (108) eingebracht wird, wobei die organische Phase (300) insbesondere mit einer laminaren Strömung in den ersten Spalt (108) eingebracht wird.

6. Verfahren (200) gemäß einem der vorangegangenen Ansprüche, bei dem im Schritt (206) des Spülens eine Spülflüssigkeit in den ersten Spalt (108) eingebracht wird, wobei die Spülflüssigkeit insbesondere mit einer laminaren Strömung in den ersten Spalt (108) eingebracht wird.

7. Verfahren (200) gemäß einem der vorangegangenen Ansprüche, bei dem im Schritt (210) des Sequenzierens eine elektrische Spannung zwischen einem den ersten Spalt (108) begrenzenden ersten Substrat (116) und einem den zweiten Spalt (110) begrenzenden zweiten Substrat (118) angelegt wird, um die DNA (500) in der Pore (114) zu sequenzieren.

## Claims

1. Method (200) for operating a microfluidic sequencing device (100) having at least one microfluidic sequencing channel (102) which fluidically connects a first microfluidic gap (108) with a second microfluidic gap (110), wherein the sequencing channel (102) is formed as a cavity (112) in the region of the first gap (108) and is formed as a pore (114) in the region of the second gap (110), wherein the pore (114) has a smaller cross section than the cavity (112), wherein the method (200) comprises the following steps:
feeding (202) a sample solution (300) into the first gap (108) in order to introduce a cell (302) into the cavity (112);
lysing (204) the cell (302) into its cellular constituents in order to release cellular DNA (500) of the cell (302);
rinsing (206) the first gap (108) in order to remove undesired cellular constituents of the cell (302) from the cavity (112) and/or the first gap (108) and to isolate the DNA (500) in the cavity (112);
filling (208) the first gap (108) and the second gap (110) with a sequencing buffer (600); and
sequencing (210) the DNA (500) in the pore (114).

2. Method (200) according to Claim 1, in which, in the feeding step (202), an aqueous solution (300) containing cells (302) is fed in as sample solution (300).

3. Method (200) according to any of the preceding claims, in which, in the feeding step (202), a centrifugation, a sedimentation and/or a vacuum treatment is further carried out in order to introduce the cell (302) into the cavity (112).

4. Method (200) according to any of the preceding claims, in which, in the lysis step (204), a chemical lysis, an enzymatic lysis, an electrical lysis, an ultrasonic lysis and/or a thermal lysis is carried out in order to release the cellular DNA (500).

5. Method (200) according to any of the preceding claims, in which, in the lysis step (204), an organic phase (300) is introduced into the first gap (108), wherein the organic phase (300) is introduced into the first gap (108) especially by means of a laminar flow.

6. Method (200) according to any of the preceding claims, in which, in the rinsing step (206), a rinse liquid is introduced into the first gap (108), wherein the rinse liquid is introduced into the first gap (108) especially by means of a laminar flow.

7. Method (200) according to any of the preceding claims, in which, in the sequencing step (210), an electric voltage is applied between a first substrate (116) bounding the first gap (108) and a second substrate (118) bounding the second gap (110) in order to sequence the DNA (500) in the pore (114).

## Revendications

1. Procédé (200) pour l'exploitation d'un séquenceur microfluidique (100), qui comprend au moins un canal de séquençage microfluidique (102), qui relie une première fente microfluidique (108) avec une deuxième fente microfluidique (110), le canal de séquençage (102) étant configuré sous la forme d'une cavité (112) dans la zone de la première fente (108) et étant configuré sous la forme d'un pore (114) dans la zone de la deuxième fente (110), le pore (114) présentant une section transversale plus petite que la cavité (112), le procédé (200) comprenant les étapes suivantes :
l'introduction (202) d'une solution d'échantillon (300) dans la première fente (108) afin d'introduire une cellule (302) dans la cavité (112) ;
la lyse (204) de la cellule (302) en ses constituants cellulaires afin de libérer l'ADN cellulaire (500) de la cellule (302) ;
le rinçage (206) de la première fente (108) afin d'éliminer les constituants cellulaires indésirables de la cellule (302) de la cavité (112) et/ou de la première fente (108) et d'isoler l'ADN (500) dans la cavité (112) ;
le remplissage (208) de la première fente (108) et de la deuxième fente (110) avec un tampon de séquençage (600) ; et
le séquençage (210) de l'ADN (500) dans le pore (114).

2. Procédé (200) selon la revendication 1, dans lequel, à l'étape (202) de l'introduction, une solution aqueuse (300) qui contient des cellules (302) est introduite en tant que solution d'échantillon (300).

3. Procédé (200) selon l'une quelconque des revendications précédentes, dans lequel une centrifugation, une sédimentation et/ou un traitement sous vide sont en outre réalisés lors de l'étape d'introduction (202) afin d'introduire la cellule (302) dans la cavité (112).

4. Procédé (200) selon l'une quelconque des revendications précédentes, dans lequel, à l'étape (204) de la lyse, une lyse chimique, une lyse enzymatique, une lyse électrique, une lyse à ultrasons et/ou une lyse thermique sont réalisées pour libérer l'ADN cellulaire (500).

5. Procédé (200) selon l'une quelconque des revendications précédentes, dans lequel, à l'étape (204) de la lyse, une phase organique (300) est introduite dans la première fente (108), la phase organique (300) étant notamment introduite dans la première fente (108) avec un écoulement laminaire.

6. Procédé (200) selon l'une quelconque des revendications précédentes, dans lequel, à l'étape (206) du rinçage, un liquide de rinçage est introduit dans la première fente (108), le liquide de rinçage étant notamment introduit dans la première fente (108) avec un écoulement laminaire.

7. Procédé (200) selon l'une quelconque des revendications précédentes, dans lequel, à l'étape (210) de séquençage, une tension électrique est appliquée entre un premier substrat (116) délimitant la première fente (108) et un deuxième substrat (118) délimitant la deuxième fente (110) afin de séquencer l'ADN (500) dans le pore (114).
